# EUROPÄISCHE PATENTSCHRIFT

(11) **EP 1 926 169 B1**
(45) Veröffentlichungstag und Bekanntmachung des Hinweises auf die Patenterteilung: **05.08.2015**
(21) Anmeldenummer: 07033557.5
(22) Anmeldetag: 16.11.2007
(51) Int. Cl.: H01M 8/04, H01M 8/06, G01N 33/00

(54) **Brennstoffzellenanlage mit Reformer**
Fuel cell system comprising a reformer
Unité de pile a combustible avec reformeur

(30) Priorität: 22.11.2006 AT 19322006
(43) Veröffentlichungstag der Anmeldung: 28.05.2008
(73) Patentinhaber: Vaillant GmbH, 42859 Remscheid (DE)
(72) Erfinder: Badenhop, Thomas, 51688 Wipperfürth (DE); Berg, Joachim, 42859 Remscheid (DE); Götz, Klaus, 42653 Solingen (DE); Kohlhage, Jörg, 45329 Essen (DE); Kraus, Matthias, 42929 Wermelskirchen (DE); Oerder, Bodo, 42855 Remscheid (DE); Paulus, Jochen, 42655 Solingen (DE)
(74) Vertreter: Hocker, Thomas

(56) Entgegenhaltungen:
- WO-A1-2006/016533
- JP-A- 2002 358 992
- US-A- 6 013 530
- US-A1- 2002 159 939
- US-A1- 2003 031 616
- US-A1- 2006 240 296

## Beschreibung

Die Erfindung bezieht sich auf eine Brennstoffzellenanlage mit Reformer.

In Brennstoffzellen findet eine elektrochemische Reaktion statt. Hierbei reagiert Wasserstoff mit Sauerstoff zu Wasserdampf, wobei elektrische und thermische Nutzenergie entsteht.

Wasserstoff ist ein Sekundärenergieträger. Er kann in Reformern gewonnen werden. Hierbei reagieren Kohlenwasserstoffe CₙHₘ in Anwesenheit von Katalysatoren mit Luftsauerstoff O₂ und / oder Wasserdampf H₂O zu Kohlendioxyd CO₂ und molekularem Wasserstoff H₂.

Erdgas wird in öffentlichen Versorgungssystemen ein Odorierungsmittel zugesetzt, damit Leckagen am Auftreten des Geruchs des Odorierungsmittels erkannt werden können. Als Odorierungsmittel wird häufig Tetrahydrothiophen (THT, C₃H₈S) beigefügt. Weitere Odorierungsmittel sind Isopropylmercaptan, Tertiärbutylmercaptan und Ethylmercaptan. Darüber hinaus enthält Erdgas als natürliche schwefelhaltige Begleitstoffe in geringen Konzentrationen Schwefelwasserstoff, Kohlenstoffoxidsulfid, Schwefelkohlenstoff und Mercaptane.

Sowohl Brennstoffzellen, als auch Reformer enthalten Katalysatoren, welche durch Schwefelverbindungen irreversibel vergiftet werden.

Der Erfindung liegt die Aufgabe zugrunde, eine Brennstoffzellenanlage sowie ein Verfahren zum Betreiben dieser Brennstoffzellenanlage zu schaffen, bei dem eine Schwefelvergiftung der Brennstoffzelle vermieden wird.

Eine Brennstoffzellenanlage nach Stand der Technik wird in WO 2006/016533 A1 offenbart.

Erfindungsgemäß wird dies gemäß den Merkmalen des unabhängigen Vorrichtungsanspruchs 1 dadurch erreicht, dass bei einer Brennstoffzellenanlage mit einer Brennstoffzelle und einem ein- oder mehrstufigen Reformer zwischen der einzigen oder ersten Reformerstufe und der Brennstoffzelle ein Adsorbens, welches sich unter Einwirkung von Schwefelwasserstoff verfärbt, angeordnet ist. Gegenüber diesem Adsorbens befindet sich eine Vorrichtung zur Erkennung der Verfärbung des Adsorbens. Eine derartige Vorrichtung besteht vorzugsweise aus einer Lichtquelle und einer Photodiode.

Gemäß den Merkmalen des abhängigen Anspruchs 2 handelt es sich bei dem Adsorbens um Blei oder Silber.

Gemäß den Merkmalen des abhängigen Anspruchs 3 befindet sich zwischen dem Adsorbens und der Brennstoffzelle ein weiterer Schwefelfilter, so dass im Falle eines Schwefeldurchbruchs durch den ersten Schwefelfilter vermieden wird, dass der Schwefel zur Brennstoffzelle gelangt.

Gemäß den Merkmalen des abhängigen Verfahrensanspruchs 4 wird in dem Falle, in dem die Vorrichtung zur Erkennung der Verfärbung des Adsorbens eine Verfärbung erkennt, ein Warnhinweis ausgegeben und/oder die Brennstoffzellenanlage abgeschaltet. Hierdurch wird eine Beschädigung der Brennstoffzelle vermieden. Vorteilhafte Merkmale bezüglich der Erkennung des Schwefeldurchbruchs werden in Anspruch 5 beschrieben. Gemäß Anspruch 6 kann die Überwachung permanent im Betrieb oder zu bestimmten Zeitpunkten, z.B. alle 10 Betriebsminuten erfolgen.

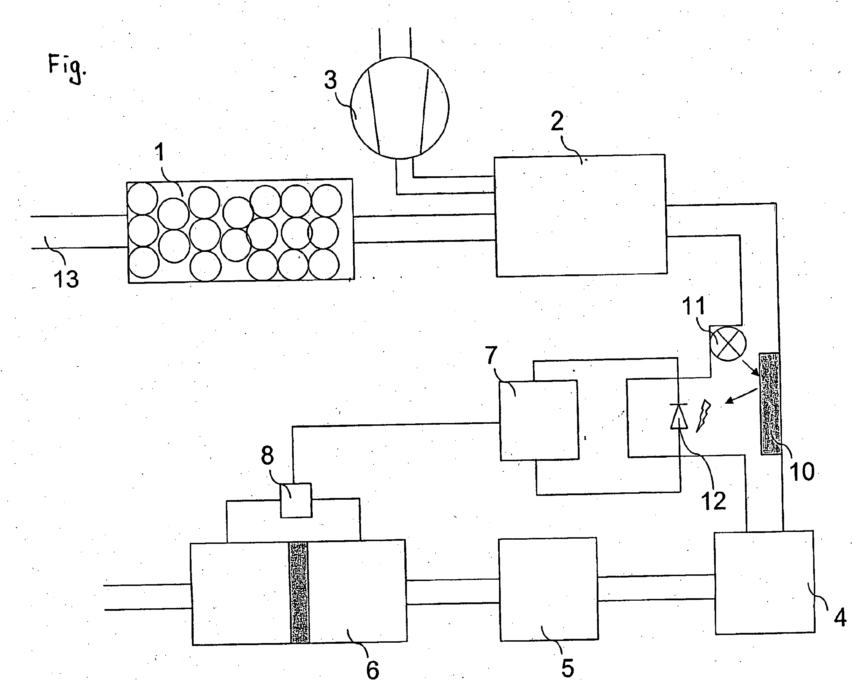

Die Erfindung wird nun anhand der Figur detailliert erläutert. Die Figur zeigt eine Brennstoffzellenanlage. Eine Erdgasleitung 13 führt zu einem ersten Schwefelfilter 1, in dem beispielsweise eine Adsorption, beispielsweise mittels Aktivkohle, an Molekularsieben oder anderen vergleichbaren Adsorptionsmitteln stattfindet. Dem Schwefelfilter 1 folgt ein Reformer 2. Dieser erhält neben gefiltertem Erdgas über ein Gebläse 3 Luft. In dem Reformer 2 wird das Erdgas, bei dem es sich um einen Kohlenwasserstoff (CₙHₘ, z.B. Methan CH₄) handelt, mit Luftsauerstoff O₂ und / oder Wasser H₂O in Anwesenheit von Katalysatoren zu Kohlendioxyd CO₂ und molekularem Wasserstoff H₂ umgewandelt. Werden schwefelhaltige Verbindungen in den Reformer 2 eingeleitet, so entsteht im Reformer 2 Schwefelwasserstoff (H₂S).

Schwefelwasserstoff bildet mit vielen Metallen Sulfide. Kommt weißliches Bleiazetat mit Schwefelwasserstoff in Berührung, so bildet sich bräunlich-schwarzes Bleisulfid

H₂S + Pb²⁺ -> PbS + 2 H⁺

Alternativ zu Bleiazetat kann Silber verwendet werden. Silber reagiert mit Schwefelwasserstoff zu schwarzem Silbersulfid.

Gegenüber dem Adsorbens 10 sind eine Lichtquelle 11 sowie eine Photodiode 12 angeordnet. Die Lichtquelle 11, beispielsweise eine Leuchtdiode, sendet Licht aus, welches an dem Adsorbens 10 reflektiert wird und auf die Photodiode 12 fällt. Das auf die Photodiode 12 einfallende Licht führt zu einem Stromfluss durch die Photodiode 12. Die Photodiode 12 ist mit einer Auswerteelektronik 7 verbunden. Stromab der Vorrichtung 9 zum Erkennen der Verfärbung des Adsorbens 10 befindet sich ein zweiter Schwefelfilter 4, eine zweite Reformerstufe 5 sowie eine Brennstoffzelle 6, welche mit einem Inverter 8 verbunden ist. Der Inverter 8 ist ferner mit der Auswerteelektronik 7 verbunden.

Wird der gesamte Schwefel aus dem Erdgas im Schwefelfilter 1 herausgefiltert, so entsteht im Reformer 2 kein Schwefelwasserstoff. Dementsprechende verfärbt sich das Adsorbens 10 nicht. Licht von der Lichtquelle 11 wird an dem Adsorbens 11 reflektiert und trifft auf die Photodiode 12. Die Auswerteelektronik 7 registriert den Stromfluss durch die Photodiode 12.

Ist der Schwefelfilter 1 beladen und kann nicht mehr den gesamten Schwefel des Erdgases zurückhalten, so bildet sich im Reformer 2 Schwefelwasserstoff. Dieser gelangt zum Adsorbens, welcher sich verfärbt. Licht von der Lichtquelle 11 wird nur noch zum Teil oder gar nicht mehr an dem Adsorbens 10 reflektiert. Als Folge dessen nimmt der Strom, welcher durch die Photodiode 12 fließt, ab oder kommt gänzlich zum Erliegen. Dies wird von der Auswerteelektronik 7 realisiert, welche bei Unterschreitung eines absoluten oder relativen (aktueller Wert zu Maximalwert) Schwellwertes ein Signal an den Inverter 8 abgibt, damit dieser die Brennstoffzellenreaktion zurückfährt. Gleichzeitig wird der Fluss von frischem Erdgas durch den Schwefelfilter 1 und die anschließende Brennstoffzellenanlage vermieden, in dem ein nicht dargestelltes Absperrventil abgeregelt wird.

## Patentansprüche

1. Brennstoffzellenanlage mit mindestens einer Brennstoffzelle (6) und einem ein- oder mehrstufigen Reformer (2, 5) zum Umwandeln von Erdgas in ein molekularen Wasserstoff führendes Prozessgas mit einem Schwefelfilter (1) stromauf des ersten Reformers (2), **dadurch gekennzeichnet, dass** zwischen der einen oder der ersten Reformerstufe (2) und der mindestens einen Brennstoffzelle (6) ein Adsorbens (10), welches sich unter Einwirkung von Schwefelwasserstoff verfärbt, und eine Vorrichtung (9) zum Erkennen der Verfärbung des Adsorbens (10), vorzugsweise eine Lichtquelle (11) und eine Photodiode (12), angeordnet sind.

2. Brennstoffzellenanlage nach Anspruch 1, **dadurch gekennzeichnet, dass** das Adsorbens (10) Bleiazetat (Pb²⁺) oder Silber (Ag) ist.

3. Brennstoffzellenanlage nach Anspruch 1 oder 2, **dadurch gekennzeichnet, dass** zwischen Adsorbens (10) und der mindestens einen Brennstoffzelle (6) ein Schwefelfilter (4) angeordnet ist.

4. Verfahren zum Betreiben einer Brennstoffzellenanlage nach einem der Ansprüche 1 bis 3, **dadurch gekennzeichnet, dass** die Vorrichtung (9) zum Erkennen der Verfärbung des Adsorbens (10) mit einer Auswerteelektronik (7) verbunden ist und beim Erkennen einer Verfärbung ein Warnhinweis ausgegeben und / oder die Brennstoffzellenanlage abgeschaltet wird.

5. Verfahren zum Betreiben einer Brennstoffzellenanlage nach Anspruch 4, **dadurch gekennzeichnet, dass** die Verfärbung des Adsorbens (10) mittels einer gegenüberliegenden Lichtquelle (11) und einer gegenüberliegenden Photodiode (12) gemessen wird und bei Unterschreitung eines vorgegebenen Ausgangssignals der Photodiode (12) oder bei Unterschreitung eines bestimmten Quotienten aus dem aktuellen Ausgangssignals der Photodiode (12) zum früher gemessenen maximalen Ausgangssignal der Photodiode (12) ein Warnhinweis ausgegeben und / oder die Brennstoffzellenanlage abgeschaltet wird.

6. Verfahren zum Betreiben einer Brennstoffzellenanlage nach Anspruch 4 oder 5, **dadurch gekennzeichnet, dass** das Verfahren permanent oder zu bestimmten Zeitpunkten durchgeführt wird.

## Claims

1. Fuel cell system having at least one fuel cell (6) and one single- or multi-stage reformer (2, 5) for the conversion of natural gas into a process gas containing molecular hydrogen using a sulphur filter (1) upstream of the first reformer (2), **characterised in that** an adsorbent (10), which changes colour under the influence of hydrogen sulphide, and a device (9) for the detection of discolouration of the adsorbent (10), preferably a light source (11) and a photodiode (12), are arranged between one or the first reformer stage (2) and the at least one fuel cell (6).

2. Fuel cell system according to claim 1, **characterised in that** the adsorbent (10) is lead acetate (Pb²⁺) or silver (Ag).

3. Fuel cell system according to claim 1 or 2, **characterised in that** a sulphur filter (4) is arranged between the adsorbent (10) and the at least one fuel cell (6).

4. Method for the operation of a fuel cell system according to one of claims 1 to 3, **characterised in that** the device (9) for the detection of discolouration of the adsorbent (10) is connected to an electronic evaluation unit (7) and upon detection of a discolouration, a warning is issued and/or the fuel cell system is switched off.

5. Method for the operation of a fuel cell system according to claim 4, **characterised in that** the discolouration of the adsorbent (10) is measured by means of an opposite light source (11) and an opposite photodiode (12) and, in the case of a predetermined output signal of the photodiode (12) being fallen below or in the case of a determined quotient from the current output signal of photodiode (12) being fallen below with respect to the previously measured maximum output signal of the photodiode (12), a warning is issued and/or the fuel cell system is switched off.

6. Method for the operation of a fuel cell system according to claim 4 or 5, **characterised in that** the method is carried out permanently or at specified intervals.

## Revendications

1. Système de cellules à combustible avec au moins une cellule à combustible (6) et un reformeur à un ou plusieurs niveaux (2, 5) destiné à convertir le gaz naturel en un gaz de traitement contenant de l'hydrogène moléculaire avec un filtre à soufre (1) en amont du premier reformeur (2), **caractérisé en ce qu'**un adsorbant (10), qui change de couleur sous l'effet de l'hydrogène sulfuré, et un dispositif (9) destiné à reconnaître le changement de couleur de l'adsorbant (10), de préférence une source lumineuse (11) et une photodiode (12), sont disposés entre l'un des niveaux ou le premier niveau du reformeur (2) et au moins une cellule à combustible (6).

2. Système de cellules à combustible selon la revendication 1, **caractérisé en ce que** l'adsorbant (10) est de l'acétate de plomb (Pb²⁺) ou d'argent (Ag).

3. Système de cellules à combustible selon la revendication 1 ou 2, **caractérisé en ce qu'**un filtre à soufre (4) est disposé entre l'adsorbant (10) et au moins une cellule à combustible (6).

4. Procédé d'exploitation d'un système de cellules à combustible selon l'une quelconque des revendications 1 à 3, **caractérisé en ce que** le dispositif (9) destiné à reconnaître le changement de couleur de l'adsorbant (10) est relié à un système électronique d'évaluation (7) et **en ce qu'**un avertissement est émis et/ou le système de cellules à combustible est arrêté lorsqu'un changement de couleur est reconnu.

5. Procédé d'exploitation d'un système de cellules à combustible selon la revendication 4, **caractérisé en ce que** le changement de couleur de l'adsorbant (10) est mesuré au moyen d'une source lumineuse opposée (11) et d'une photodiode opposée (12) et **en ce que**, en cas de dépassement de la limite inférieure d'un signal de sortie prédéfini de la photodiode (12) ou en cas de dépassement de la limite inférieure d'un quotient déterminé en dehors du signal de sortie actuel de la photodiode (12) par rapport au précédent signal de sortie maximum mesuré de la photodiode (12), un avertissement est émis et/ou le système de cellules à combustible est arrêté.

6. Procédé d'exploitation d'un système de cellules à combustible selon la revendication 4 ou 5, **caractérisé en ce que** le procédé est exécuté de façon permanente ou ponctuelle.
